Europäisches Patentamt

European Patent Office

Office européen des brevets

(19)

(11) Publication number: **0 132 998**
**B1**

## EUROPEAN PATENT SPECIFICATION

(12)

(45) Date of publication of patent specification: 28.01.87

(21) Application number: 84304959.4

(22) Date of filing: 20.07.84

(51) Int. Cl.⁴: **C 12 N 11/08**

(54) A process for the production of enzyme fixed resin material and a method for the regeneration thereof.

(30) Priority: 29.07.83 JP 138774/83

(43) Date of publication of application:
13.02.85 Bulletin 85/07

(45) Publication of the grant of the patent:
28.01.87 Bulletin 87/05

(84) Designated Contracting States:
DE FR GB NL

(56) References cited:
DD-A- 153 130
DE-A-2 513 412
GB-A-1 492 937
GB-A-2 082 188

(73) Proprietor: KYOWA HAKKO KOGYO CO., LTD.
6-1, Ohte-Machi 1-chome
Chiyoda-ku Tokyo-to (JP)

(72) Inventor: Nagashima, Minoru
1-14-46, Keigo-machi
Hofu-shi Yamaguchi-ken (JP)
Inventor: Azuma, Masaki
2-3-303, Kyowa-cho
Hofu-shi Yamaguchi-ken (JP)
Inventor: Noguchi, Sadao
2542, Oaza-Fujimagari
Ube-shi Yamaguchi-ken (JP)

(74) Representative: Lambert, Hugh Richmond et al
D. YOUNG & CO. 10 Staple Inn
London, WC1V 7RD (GB)

Courier Press, Leamington Spa, England.

# Description

This invention relates to a process for production of an enzyme fixed resin material and a method for regeneration thereof.

Heretofore, in producing a fixed enzyme by an adsorption method, an anion or cation exchange resin of a macro-porous type or a macro-recticular structure has been employed, but such a resin has a number of problems.

For example, in a process employing Duolite A7 which is a polyphenol type weakly basic anion exchange resin (Japanese Published Unexamined Patent Application 80160/1974), elution is difficult and, hence, regeneration is difficult because adsorption is strong. On this ground, it is proposed to weaken the adsorption due to the hydrophobic properties by further introducing hydrophilic groups (Japanese Published Unexamined Patent Application 85890/1978), but this proposal is not adequate.

On the other hand, in a method employing Amberlite IRA—904 which is a styrene-divinylbenzene type weakly basic anion exchange resin (Japanese Published Unexamined Patent Application 53584/1975), the enzyme is generally loosely adsorbed and gradually comes off during use, and also the preferential adsorption of poly-saccarides occurs (Japanese Published Unexamined Patent Application 147992/1979).

In a continuous method which employs a fixed enzyme, contamination with infectious microbes in a reactor often becomes a problem and, as countermeasures therefor, conditions of a high substrate concentration and medium to high temperatures are employed. However, the countermeasures in turn often cause the deterioration of the resin (for example, obstruction of the contact of a substrate with the enzyme due to polysaccharides irreversibly adsorbed, etc.).

Under the above circumstances, it has been found in accordance with this invention that it is possible to obtain an enzyme-fixed resin material which can stand continuous use as medium to high temperature and can be regenerated and reused by selecting a styrene-divinylbenzene type basic anion exchange resin which is resistant to deterioration and on which reversible adsorption occurs in general and by overcoming the drawback of weak adsorption by crosslinking with a polyvalent aldehyde.

The polyvalent aldehyde crosslinks enzymes by chemically combining with functional groups, especially amino groups in the enzymes. In this aspect, it is necessary that any functional group containing nitrogen does not contain active hydrogen.

The present invention is described in more detail hereinafter.

As the styrene-divinylbenzene type basic anion exchange resin having a macro-reticular structure and wherein any ion exchange group containing nitrogen does not have active hydrogen, those having the mean macropore radius of 100—2000 Å (0.01 to 0.2 μm) especially 200—1000 Å (0.02 to

0.1 μm) and the macropore volume of 0.1 ml/g or more, especially 0.2—1.0 ml/g, for example, Imac A20S, A20R [tradenames by Duolite International Co. (France)], Lewatit MP500A [tradename by Bayer Co. (West Germany)], Duolite A378, A369, A368 [tradenames by Diamond Shamrock Co. (U.S.A.)], and Diaion HPA75, HPA25 [tradenames by Mitsubishi Chemical Industries Co. Ltd (Japan)] can be preferably employed.

Polyvalent aldehydes to be used are not specifically limited, but preferably include saturated aliphatic dialdehydes having 2—10 carbon atoms such as glyoxal, malonaldehyde, succinaldehyde, glutaraldehyde and adipoaldehyde.

Enzymes to be used in the present invention are not specifically limited, and include oxidoreductase, transferase, hydrogenase, lyase, isomerase, ligase, and the like. In particular, glucose isomerase, glucoamylase, fumarase, aspartase, lipase, invertase, protease, urease, amino acid oxidase, etc., are especially suitable.

The production of the enzyme-fixed resin material of the present invention is described hereinafter. The present enzyme-fixed resin material may be obtained by bringing a styrene-divinylbenzene type basic anion exchange resin which has a macro-reticular structure and wherein any nitrogen-containing ion exchange group does not have active hydrogen into contact with a solution containing an enzyme to cause the enzyme to be adsorbed on the resin, then bringing the resin into contact with a solution of a substrate for said enzyme and further bringing the resin into contact with a polyvalent aldehyde solution.

As the solution containing the enzyme, there may be employed a microbial cell fracture liquor or its filtrate, a solution of a crude enzyme in water or a buffer solution, the crude enzyme being obtained by precipitation with ammonium sulfate or with a solvent from the filtrate, a solution obtained by further dialyzing the solution, a purified enzyme solution, etc.

Suitable range of concentration of the enzyme solution is 0.01—200 mg/l as protein.

The amount of the resin to be used may be prescribed as the amount of the enzyme to be adsorbed, and is generally suitably in the range of 0.1—100 mg-protein/ml-resin.

The adsorption of the enzyme is conducted generally in a batchwise operation with stirring at 0—40°C for 30 minutes to 72 hours. Alternatively, the adsorption may be conducted by passing an enzyme solution through a column packed with a resin.

The pH is maintained within a pH region where the enzyme is stable, generally at 4—10, and the pH adjustment is preferably done with a buffer solution.

Thereafter, with or without solid-liquid separation, the resin is brought into contact with a solution of a substrate for the enzyme. This treatment is for preventing the active centers from being affected in the subsequent treatment with the polyvalent aldehyde, by temporarily combining the active centers with the substrate. The contact may be conducted batchwise or in a column similarly as the adsorption of the enzyme.

As the substrate, substrates for the respective enzymes may be employed and, for example, fumaric acid, malic acid, sodium, ammonium and potassium salts thereof, etc., for fumarase; glucose, fructose, etc., for glucose isomerase; and soluble starch, maltose, glucose, maltotriose, etc., for glucoamylase are employed. As the substrate solution, an aqueous solution, a buffer solution, etc., of the substrate may be employed.

The concentration of the substrate at the contact is 0.1 mM—1.0 M, and where the substrate is an electrolyte, it is preferred that the concentration is 0.2 M or less in order to prevent the desorption of the enzyme due to the electrolyte.

The amount of the substrate to be used is suitably in the range of about 10—1,000 times the molar amount of polyvalent aldehyde to be used, considering that the substrate protects the active center in competition with the polyvalent aldehyde.

The treating temperature, pH, etc., may be similar to those of the adsorption of the enzyme, and the time may vary from about 10 minutes to 10 hours depending on the amounts of the substrate and the polyvalent aldehyde; generally the time is 30—60 minutes.

Thereafter, the mixture is brought into contact with the polyvalent aldehyde solution without solid-liquid separation. The method for contact may be similar to that in the substrate treatment. The polyvalent aldehyde solution is generally an aqueous solution of a polyvalent aldehyde or, where sparingly soluble in water, methanol, ethanol, or the like solution, and the concentration at contact is suitably 1—100 mM. The amount of the polyvalent aldehyde used is in the range of 0.001 mM to 1 mM per mg of the protein.

The treatment with the polyvalent aldehyde is generally conducted at 0—40°C for one minute to 48 hours. Further, it is preferred to keep the pH constant in the stable pH range of the enzyme by using a buffer solution, etc.

After the treatment with the polyvalent aldehyde, the resin is washed with a buffer solution, water, methanol, ethanol, etc., to remove the unreacted polyvalent aldehyde, the unreacted protein, etc., whereby an enzyme-fixed resin material is obtained.

The present enzyme-fixed resin material manifests excellent activity and causes substantially no side reactions. Further, the present resin material is improved in heat resistance and, for example, the activity can be maintained even in continuous use at 40—70°C for a long period, e.g. for at least 3—10 months in case of the fumarase-fixed resin material.

Furthermore, the resin can be regenerated by bringing the enzyme-fixed resin material deteriorated with use for a certain period into contact with an aqueous mineral acid solution containing 0.2—5 M salt to rupture the crosslinks by the polyvalent aldehyde and desorb the enzyme.

As the salt, a strong, alkali salt or weak alkali salt of a strong acid, especially a strong alkali salt, is preferred. For example, alkali metal salts (e.g., sodium chloride, sodium sulfate, etc.), alkaline earth metal salts (e.g., calcium chloride, etc.), and ammonium salts (e.g. ammonium chloride, etc.) of sulfuric acid, hydrochloric acid, phosphoric acid, etc., are employed.

As the mineral acid, sulfuric acid, hydrochloric acid, phosphoric acid, etc., are employed.

As the mode of contact, a batchwise mode with stirring, passing a liquor for regeneration through a resin material packed in a column, etc., is applied.

The operation at the contact is suitably conducted at a mineral acid concentration of 0.5—6 N and 40—95°C.

By subjecting the regenerated resin to an enzyme fixing operation similar to the above, an enzyme-fixed resin material may be obtained, and the activity and characteristics of the material are maintained at nearly the same level as in the case where a new resin is employed.

Further, similar characteristics may be maintained also after twice or three times of regeneration and reuse.

As regards the regeneration and reuse of the carrier, there is a method by electrolytic salt aqueous solution treatment and acid treatment (Japanese Published Unexamined Patent Application 70871/1976), but the regeneration by cutting the crosslinks requires treatment with an aqueous mineral acid solution containing a salt, and thus the regenerating method of the present invention is fundamentally different from the method described in the above laid-open patent application.

The present invention is described in more detail below referring to the following examples.

Example 1

At first, 300 ml of a medium containing 10% glucose, 0.5% potassium dihydrogenphosphate, 0.6% urea, 0.05% magnesium sulfate (heptahydrate), 2% peptone, 0.05% meat extract and 50 γ/l biotin is charged into a 2,000 ml-Erlenmeyer flask, and a fumarase-producing microorganism, *Corynebacterium glutamicum* ATCC 21513 is inoculated and cultured, with shaking, at a culture temperature of 30°C for 48 hours. The microbial cells are collected from the culture liquor, washed, and freeze-dried to obtain 9 g of the microbial cells. The microbial cells are dispersed in a 0.1 M phosphate buffer (pH 6.0) at a concentration of 50 g/l, and subjected to ultrasonic treatment.

As the carriers, there are used Imac A20S (Duolite International Co., France) as the carrier of the present invention and Duolite A6 (Diamond Shamrock Co., U.S.A.) as a control. Each carrier is washed thoroughly with deionized water and, thereafter, buffered with a 0.1 M phosphate buffer (pH 6.0). Thereafter, the crude enzyme suspension prepared above is brought into contact with the resin at a proportion of 200 U (as the activity value) per ml of the resin at room temperature for 16 hours. The amount of the protein adsorbed is 90% or more in either case. Thereafter, an equal amount to that of the resin of a 0.1 M aqueous sodium fumarate solution is added thereto as a stabilizer for the enzyme. The supernatent is discarded, and then an equal amount to that of the resin of a 0.2% aqueous glutaraldehyde solution is added to the resin to conduct the reaction at room temperature for 30 minutes.

Each resin is thoroughly washed to obtain fixed enzyme carriers A (Imac A20S) and B (Duolite A6), respectively. Each fixed enzyme is filled into a 10 ml-jacketed column, and a 1 M aqueous sodium fumarate solution is passed through the column at 50°C at a space velocity of SV = 0.3 (feed vol./carrier-vol.·hr). Conversions to malic acid of about 80% with the carrier A and about 78% with the carrier B are obtained over 20 days, which demonstrate that a continuous production is possible.

### Example 2

When operations are continued with the carriers of Example 1 for another month, conversions are reduced to about 78% with the carrier A and about 75% with the carrier B, but there is no contamination due to infectious microbes. Further, the resin is pulled out from the column, 0.5 N sulfuric acid containing 0.5 M sodium chloride is added to the resin, and the mixture is heated at 70°C and maintained at the same temperature for 2 hours. The resin is washed with deionized water, and an enzyme is again fixed thereto in the same manner as in Example 1. Namely, the crude enzyme suspension is prepared in the same manner as in Example 1, and the enzyme suspension is brought into contact with the resin at a proportion of 200 U per ml of the resin to adsorb the enzyme. The amounts of the protein adsorbed are 90% with the carrier A and 40% with the carrier B. Similarly, a sodium fumarate solution is added thereto, and crosslinking is conducted using glutaraldehyde in the same manner as in Example 1 to fix the enzyme. The fixed enzyme carriers A and B are thoroughly washed and filled into 10 ml-columns, respectively, and a 1 M aqueous sodium fumarate solution is passed through each column at Sv = 0.3. Conversion to malic acid in the effluent can be maintained at 80% for 20 days with the carrier A; whereas it rapidly drops to 25% on the first day, to 15% on the 10th day, and to 11% on the 20th day with the carrier B.

Further, regeneration and reuse of the fixed enzyme carrier A are repeated in the same manner as above, and as the result, conversion to malic acid in the effluent can be maintained at 80% for 20 days both in the second and third reuses.

### Example 3

At first, 25 g of wet microbial cells of *Streptomyces phaeochromogenes* IFO 3105 are autodigested in a 0.02 M phosphate buffer (pH 8), and thereafter the mixture is filtered to obtain a glucose isomerase enzyme solution.

Duolite A378 (Diamond Shamrock Co., U.S.A.) buffered to pH 8 is suspended in the enzyme solution, and the suspension is adjusted to pH 8.2, and thereafter mildly stirred at room temperature for about 16 hours.

Thereafter, the supernatent is discarded, and 50 ml of a 1% aqueous glucose solution is added to the resin. The mixture is gently stirred for 10 minutes, and then the supernatant is discarded. Then, 50 ml of a 0.2% aqueous glutaraldehyde solution is added to the resin, and the mixture is gently stirred at room temperature for 30 minutes to effect crosslinking.

The resin is charged into a column and thoroughly washed with water. A 0.5 M glucose solution containing 0.05 M $MgSO_4$ and $5 \times 10^{-4}$ M $CoCl_2$ is passed through the insoluble enzyme column at a rate of 500 ml per hour (SV = 10) at 60°C. The fructose in the effluent is 30 mg/ml by the cystein-carbazole method.

When passed at SV = 4, the fructose is 46 mg/ml and the glucose is 44 mg/ml by the glucose oxidase method, and thus a degree of isomerization of 51% is obtained. Further, when the sugar solution is continuously passed through the column at SV = 4 for 20 days, the degree of isomerization is 49% on the 20th day. Then, the resin is pulled out from the column and thoroughly washed with water, and 0.5 N sulfuric acid containing 0.5 M sodium chloride is added thereto. The mixture is heated at 70°C for 2 hours. The resin is washed, and refixing is conducted in the same manner as in the first fixing operation. When the same sugar solution as above is passed through the refixed carrier at SV = 10, the fructose in the effluent is 31 mg/ml, from which it is apparent that the refixed resin exhibits the same degree of activity as the new resin.

### Example 4

Glucoamylase (produced by Nagase Sangyo Co., Ltd.) is dissolved in a 0.1 M acetate buffer of pH 4.5 to prepare a solution containing 14 mg of the enzyme as protein per ml. Then, 10 ml of the solution is added to 5 ml of HPA75 resin (produced by Mitsubishi Chemical Industries Co., Ltd.), and the mixture is shaken in a thermostat of 37°C for 16 hours, whereby 22 mg of the protein is adsorbed per ml of the resin.

Further, the resin is washed, the supernatent is discarded, 5 ml of a 1% aqueous soluble starch solution is added to the resin, and the mixture is gently stirred. Then, the supernatent is discarded, 5 ml of a 0.2% aqueous glutaraldehyde solution is added to the resin, and the mixture is gently stirred at room temperature for 30 minutes to effect crosslinking.

The resin is transferred to a column, thoroughly washed with water, and a 10% aqueous soluble starch solution is passed through the insoluble enzyme column at SV = 1.0 at 50°C. The glucose concentration in the effluent is 85 g/l on the third day, and 83 g/l on the 10th day.

Further, when passed through the column for 50 days, the glucose concentration is somewhat reduced to 80 g/l, but contamination with infectious microbes is not remarkably observed.

Further, the resin is pulled out and washed with water, and then 0.5 N sulfuric acid containing 0.5 M sodium chloride is added to the resin. The mixture is heated at 70°C for 2 hours. The resin is washed, and refixing is conducted in the same manner as in a new resin. The obtained resin is transferred to a column, and a 10% aqueous soluble starch solution is passed through the insoluble enzyme column at SV = 1.0 at 50°C. The glucose concentration in the effluent is 85 g/l on the third day, and 83 g/l on the 10th day.

As a control, the above-described fixing operation is repeated using a Duolite A7 resin. The obtained enzyme-fixed resin is filled in a column, and a 10% aqueous soluble starch solution is passed at SV = 1.0 at 50°C. The glucose concentration in the effluent is 80 g/l on the third day, and 78 g/l on the 10th day. Further, on the 20th day, it is reduced down to 40 g/l. Thereafter, regeneration and refixing are effected similarly as in the above-described example. The glucose concentration in the effluent in the case where the refixed carrier is used cannot give results satisfactory for reuse; i.e., 30 g/l on the third day, and 25 g/l on the 10th day. Further, an enzyme-fixed Duolite A7 resin is prepared in the same manner as above, and a 10% aqueous soluble starch solution is passed at SV = 1.0 at 40°C. In this case, contamination with infectious microbes gradually proceeds, and the glucose concentration is reduced to 75 g/l on the third day, and to 60 g/l on the 10th day. Similar results are obtained when Imac A20SU resin is used in place of Duolite A7 resin in the same procedure under the same conditions as above.

Further, comparison with a fixing method employing a Duolite A7 resin, but not employing glutaraldehyde is made hereinafter. Namely, 10 ml of the Duolite A7 resin is mixed with 20 ml of a glucoamylase solution of a concentration of 10 mg/ml as a protein prepared using a 0.1 M acetate buffer (pH 4.5). The mixture is shaken at 37°C for 16 hours to effect fixing of the enzyme, whereby 23 mg of the protein is adsorbed. Each 5 ml of the resin is filled in two columns and washed, and a 10% aqueous soluble starch solution is passed through each insoluble enzyme column at SV = 1.0. One column is maintained at a temperature of 50°C (Experiment A), and the other at 40°C (Experiment B). In case of Experiment A, the glucose concentration in the effluent is 78 g/l on the third day, and 65 g/l on the 10th day, which reveals large drop of the enzyme activity. In case of Experiment B, the glucose concentration in the effluent is reduced to 73 g/l on the third day and to 58 g/l on the 10th day, and also contamination with infectious microbes is observed.

Regeneration and refixing are conducted using both carriers in Experiments A and B. The regenerating conditions are a method of heating the used resin in 0.5 N sulfuric acid containing 0.5 M sodium chloride at 70°C (Experiments A—1 and B—1) and a method of heating the used resin in 4 N sodium hydroxide at 60°C (Experiments A—2 and B—2). The regenerated resins are refixed in the same manner as in the first fixing. The amounts of the protein absorbed are 10 mg in A—1, 10 mg in B—1, 15 mg in A—2 and 13 mg in B—2, and thus the adsorption ability is reduced as compared in the first time. When the resins are filled into columns and a 10% aqueous soluble starch solution is passed through each column at SV = 1.0 at 40°C, the glucose concentrations in the effluent are merely 20 mg/ml in A—1, 18 mg/ml in B—1, 25 mg/ml in A—2, and 20 mg/ml in B—2 on the third day, respectively.

Example 5

The same procedure as in Example 1, is repeated except that Lewatit MP 500 A is used in place of Imac A 20 S and almost the same result as therein is obtained.

Claims

1. A method of preparing an immobilized enzyme composition comprising a macroreticular styrene-divinylbenzene basic anion exchange resin having an ezyme adsorbed therein and which comprises contacting the basic anion exchange resin, being a resin in which any nitrogen-containing ion exchange groups are free of active hydrogen atoms, with a solution containing the enzyme, thereby to adsorb the enzyme onto the resin, and thereafter cross-linking the enzyme *in situ* by reaction with a polyvalent aldehyde, characterised in that, prior to cross-linking with the polyvalent aldehyde, the macroreticular ion exchange resin containing the adsorbed enzyme is contacted with a substrate for the enzyme thereby to temporarily block reactive sites on the enzyme during the cross-linking step.

2. A process according to claim 1, wherein said styrene-divinylbenzene type basic anion exchange resin has a mean macropore radius of 100—2000Å (0.01 to 0.2 μm) and a macropore volume of 0.1 ml/g or more.

3. A process according to claim 2, wherein said styrene-divinylbenzene type basic anion exchange resin has a mean macropore radius of 200—1000Å (0.02 to 0.1 μm) and a macropore volume of 0.2—1.0 ml/g.

4. A process according to claim 1, 2 or 3, wherein said styrene-divinylbenzene type basic anion exchange resin is Imac A20S, A20R, Duolite A378, A369, A368, Lewatit MP500A, Diaion HPA75 or HPA25.

5. A process according to claim 1, 2, 3 or 4, wherein the enzyme is an oxidoreductase, transferase, hydrogenase, lyase, isomerase or ligase.

6. A process according to claim 5, wherein the enzyme is a glucose isomerase, glucoamylase, fumarase, aspartase, lipase, invertase, protease, urease or amino acid oxidase.

7. A process according to any one of claims 1—6, wherein the enzyme is adsorbed onto the resin by contacting the resin with i) a microbial cell fracture liquor or its filtrate; ii) a solution of a crude enzyme in water or a buffer solution, the crude enzyme being obtained by precipitation with ammonium sulfate or with a solvent from the filtrate; iii) a solution obtained by dialyzing a solution as defined under ii) or iv) a purified enzyme solution.

8. A process according to any one of claims 1—7, wherein the resin is contacted with an enzyme solution containing from 0.01—200 mg/l of enzyme calculated as protein.

9. A process according to any one of claims 1—8, wherein the amount of the enzyme, calculated as protein, adsorbed onto the resin is in the range of 0.1—100 mg/ml-resin.

10. A process according to any one of claims 1—9, wherein the adsorption of the enzyme onto the resin is conducted in a batchwise operation with stirring at 0—40°C for 30 minutes to 72 hours.

11. A process according to any one of claims 1—10, wherein the adsorption of the enzyme onto the resin is conducted by passing the enzyme solution through a column packed with said resin.

12. A process according to any one of claims 1—11, wherein, prior to contact with the polyvalent aldehyde, the resin containing the adsorbed enzyme is contacted with a solution of a substrate for the enzyme in which the concentration of the substrate at contact is 0.1 mM-1.0 M.

13. A process according to any one of claims 1—12, wherein the amount of the substrate used is in the rnage 10—1,000 times the molar amount of the polyvalent aldehyde subsequently used in the cross-linking step.

14. A process according to any one of claims 1—13, wherein the treatment with the substrate solution is conducted with stirring at 0—40°C for 10 minutes to 10 hours.

15. A process according to any one of the preceding claims, wherein the polyvalent aldehyde used in the cross-linking step is a saturated aliphatic dialdehyde having 2—10 carbon atoms.

16. A process according to any one of the preceding claims, wherein the cross-linking step is effected by contacting the resin containing the adsorbed enzyme with a solution containing the polyvalent aldehyde in which the concentration of the polyvalent aldehyde at contact is 1—100 mM.

17. A process according to any one of the preceding claims, wherein the amount of the polyvalent aldehyde used is in the range of 0.001 mM to 1 mM per mg of the enzyme, calculated as protein.

18. A process according to any one of the preceding claims, wherein the treatment with the polyvalent aldehyde is conducted at 0—40°C for one minute to 48 hours.

19. A method of regenerating an immobilised enzyme composition, being a composition produced by a method as claimed in any one of claims 1—18, which comprises treating the immobilised enzyme composition, after use, with an aqueous mineral acid solution containing an alkaline salt of mineral acid at a concentration of from 0.2 to 5 M, thereby rupturing the bonds cross-linking the enzyme and desorbing the enzyme from the resin, and adsorbing fresh enzyme into the resin by a method claimed in any one of claims 1—18.

20. A method according to claim 19, wherein the aqueous acid solution used to rupture the cross-linking bonds and desorb the enzyme comprises a mineral acid at a concentration of 0.5—6 N.

21. A method according to claim 20 or 21, wherein the alkaline salt is an alkali metal, alkaline earth metal or ammonium salt of sulfuric acid, hydrochloric acid or phosphoric acid, and the mineral acid is sulfuric acid, hydrochloric acid or phosphoric acid.

22. A method according to any one of claims 19—21, wherein the enzyme is desorbed by contact with said mineral acid solution at a temperature in the range 40—95°C.

**Patentansprüche**

1. Verfahren zur Herstellung eines immobilisierten Enzympräparates, umfassend ein an einem makroporösen basischen Anionenaustauscherharz auf Basis Styrol-Divinylbenzol adsorbiertes Enzym, bei dem man das basische Anionenaustauscherharz, in dem alle Stickstoff enthaltenden Austauschgruppen frei von aktivem Wasserstoff sind, zusammenbringt mit einer Lösung des Enzyms und dieses dadurch an dem Harz adsorbiert, und anschließend das Enzym in situ durch Umsetzung mit einem mehrwertigen Aldehyd vernetzt, dadurch gekennzeichnet, daß man vor der Vernetzung mit dem mehrwertigen Aldehyd das makroporöse Ionenaustauscherharz, welches das adsorbierte Enzyme enthält, mit einem Substrat für das Enzym in Kontakt bringt und dadurch reaktive Stellen auf dem Enzyme während des Vernetzungsschrittes zweitweise blockiert.

2. Verfahren nach Anspruch 1, wobei das basische Anionenaustauscherharz auf Basis Styrol-Divinylbenzol einen mittleren Makroporen-Radius von 100 bis 2000 Å (0,01 bis 0,2 μm) und ein Porenvolumen von mindestens 0.1 ml/g hat.

3. Verfahren nach Anspruch 2, wobei das basische Anionenaustauscherharz auf Basis Styrol-Divinylbenzol einen mittleren Makroporen-Radius von 200 bis 1000 Å (0,02 bis 0,1 μm) und ein Porenvolumen von 0,2 bis 1,0 ml/g hat.

4. Verfahren nach Anspruch 1, 2 oder 3, wobei das basische Anionenaustauscherharz auf Basis Styrol-Divinylbenzol aus den Typen Imac A20S, A20R, Duolite A378, A369, A368, Lewatit MP500A, Diaion HPA75 oder HPA25 besteht.

5. Verfahren nach Anspruch 1, 2, 3 oder 4, wobei das Enzym eine Oxidoreductase, Transferase, Hydrogenase, Lyase, Isomerase oder Ligase ist.

6. Verfahren nach Anspruch 5, wobei das Enzyme eine Glukoseisomerase, Glucoamylase, Fumarase, Aspartase, Lipase, Invertase, Protease, Urease oder Aminosäureoxidase ist.

7. Verfahren nach einem der Ansprüche 1 bis 6, wobei man das Enzym an das Harz adsorbiert durch zusammenbringen mit

1.) einem Homogenisat von Mikroorganismus-Sellen oder dessen Filtrat,

2.) einer Lösung eines Rohenzyms in Wasser oder einer Pufferlösung, wobei das Rohenzym aus dem Filtrat erhalten wird durch Fällung mit Ammoniumsulfat oder mit einem Lösungsmittel,

3.) einer Lösung, erhalten durch Dialyse einer Lösung gemäß Ziff. 2., oder

4.) einer Lösung eines gereinigten Enzyms.

8. Verfahren nach einem der Ansprüche 1 bis 7, wobei man das Harz zusammenbringt mit einer Enzymlösung, die 0,01 bis 200 mg/Liter Enzym enthält, berechnet als Protein.

9. Verfahren nach einem der Ansprüche 1 bis 8, wobei die Menge des an das Harz adsorbierten Enzyms, berechnet als Protein, im Bereich von 0,1 bis 100 mg/ml Harz liegt.

10. Verfahren nach einem der Ansprüche 1 bis 9, wobei man die Adsorption des Enzyms an das Harz chargenweise unter Rühren bei 0 bis 40°C während 30 Minuten bis 72 Stunden durchführt.

11. Verfahren nach einem der Ansprüche 1 bis 10, wobei man die Adsorption des Enzyms an das Harz durch Leiten der Enzymlösung durch eine mit dem genannten Harz gefüllte Säule bewerkstelligt.

12. Verfahren nach einem der Ansprüche 1 bis 11, wobei man das Harz mit dem absorbierten Enzym vor dem Zusammenbringen mit dem mehrwertigen Aldehyd mit einer Lösung eines Substrats für das Enzym in Berührung bringt, wobei die Konzentration des Substrats während des Zusammenbringens 0,1 mM bis 0,1 M beträgt.

13. Verfahren nach einem der Ansprüche 1 bis 12, wobei die Menge des verwendeten Substrats im Bereich des 10- bis 1000-fachen der molaren Menge des mehrwertigen Aldehyds liegt, der anschließend für den Vernetzungsschritt verwendet wird.

14. Verfahren nach einem der Ansprüche 1 bis 13, wobei man die Behandlung mit der Substratlösung unter Rühren bei 0 bis 40°C während 10 Minuten bis 10 Stunden durchführt.

15. Verfahren nach einem der vorhergehenden Ansprüche, wobei der für den Vernetzungsschritt verwendete mehrwertige Aldehyd ein gesättigter aliphatischer Dialdehyd mit 2 bis 10 Kohlenstoffatomen ist.

16. Verfahren nach einem der vorangegangenen Ansprüche, wobei der Vernetzungsschritt bewerkstelligt wird durch Zusammenbringen des das adsorbierte enthaltenden Harzes mit einer Lösung des mehrwertigen Aldehyds, wobei die Konzentration an mehrwertigem Aldehyd während des Zusammenbringens 1 bis 100 mM beträgt.

17. Verfahren nach einem der vorangegangenen Ansprüche, wobei die Menge des verwendeten mehrwertigen Aldehyds im Bereich von 0,001 mM bis 1 mM pro mg Enzym, berechnet als Protein, liegt.

18. Verfahren nach einem der vorangegangenen Ansprüche, wobei man die Behandlung mit dem mehrwertigen Aldehyd bei 0 bis 40°C während 1 Minute bis 48 Stunden durchführt.

19. Verfahren zur Regeneration eines nach den Verfahren der Ansprüche 1 bis 18 hergestellten immobilisierten Enzympräparates, umfassend die Behandlung des immobilisierten Enzympräparates nach Gebrauch mit einer wäßrigen Mineralsäurelösung, welche ein alkalisches Salz einer Mineralsäure in einer Konzentration von 0,2 bis 5 Mol enthält, dadurch Spalten der Bindungen, die das Enzym vernetzen, und Desorbieren des Enzyms von Harz, und anschliessend Adsorbieren von frischem Enzym an das Harz nach einem Verfahren nach einem der Ansprüche 1 bis 18.

20. Verfahren nach Anspruch 19, wobei die zur Spaltung der Vernetzungsbindungen und zur Desorption des enzyms verwendete wäßrige Säurelösung eine 0,5 bis 6 normale Mineralsäure ist.

21. Verfahren nach Anspruch 19 oder 20, wobei das alkalische Salz ein Alkali-, Erdalkali- oder Ammoniumsalz der Schwefelsäure, Salzsäure oder Phosphorsäure und die Mineralsäure Schwefelsäure, Salzsäure oder Phosphorsäure ist.

22. Verfahren nach einem der Ansprüche 19 bis 21, wobei das enzym durch Zusammenbringen mit der Säurelösung bei einer Temperatur im Bereich von 40 bis 95°C desorbiert wird.

## Revendications

1. Procédé pour la préparation d'une composition à enzyme immobilisé, comprenant une résine basique échangeuse d'anions macroréticulaire de styrène-divinylbenzène dans laquelle est adsorbée une enzyme, qui comporte la mise en contact de la résine basique échangeuse d'anions, qui est une résine dans laquelle tout groupe échangeur d'ions contenant de l'azote est exempt d'atomes d'hydrogène actif, avec une solution contenant l'enzyme, pour adsorber ainsi l'enzyme sur la résine, et ensuite la réticulation de l'enzyme *in situ* par réaction avec un polyaldéhyde, caractérisé en ce que, avant la réticulation avec le polyaldéhyde, la résine échangeuse d'ions macroréticulaire contenant l'enzyme adsorbée est mise en contact avec un substrat de l'enzyme, pour que soient ainsi bloqués temporairement les sites réactifs de l'enzyme au cours de l'étape de réticulation.

2. Procédé selon la revendication 1, dans lequel ladite résine basique échangeuse d'anions de type styrène-divinylbenzène possède un rayon moyen des macropores de 100—2000 Å (0,01 à 0,2 µm) et un volume de macropores de 0,1 ml/g ou plus.

3. Procédé selon la revendication 2, dans lequel ladite résine basique échangeuse d'anions de type styrène-divinylbenzène possède un rayon moyen de macropores de 200—100 Å (0,02 à 0,1 µm) et un volume de macropores de 0,2—1,0 ml/g.

4. Procédé selon l'une quelconque des revendi-

cations 1, 2 ou 3, dans lequel ladite résine basique échangeuse d'anions de type styrène-divinylbenzène est Imac A20S, A20R, Duolite A378, A369, A368, Lewatit MP500 A, Diaion HPA75 ou HPA25.

5. Procédé selon l'une quelconque des revendications 1, 2, 3 ou 4, dans lequel l'enzyme est une oxydoréductase, une transférase, une hydrogénase, une lyase, une isomérase ou une ligase.

6. Procédé selon la revendication 5, dans lequel l'enzyme est une glucose-isomérase, une glucoamylase, une fumarase, une aspartase, une lipase, une invertase, une protéase, une uréase ou une amino-acide-xoydase.

7. Procédé selon l'une quelconque des revendications 1—6, dans lequel l'enzyme est adsorbée sur la résine par mise en contact de la résine avec i) une liqueur de rupture de cellules microbiennes ou son filtrat; ii) une solution d'une enzyme brute dans l'eau ou une solution tampon, l'enzyme brute étant obtenue par précipitation avec du sulfat d'ammonium ou avec un solvant à partir du filtrat; iii) une solution obtenue en dialysant une solution telle que définie sous ii); ou iv) une solution purifiée d'enzyme.

8. Procédé selon l'une quelconque des revendications 1—7, dans lequel la résine est mise en contact avec une solution d'enzyme contenant de 0,01 à 200 mg/l d'enzyme calculée en protéine.

9. Procédé selon l'une quelconque des revendications 1—8, dans lequel la quantité de l'enzyme, calculée en protéine, adsorbée sur la résine est comprise dans l'intervalle de 0,1—100 mg/ml de résine.

10. Procédé selon l'une quelconque des revendications 1—9, dans lequel l'adsorption de l'enzyme sur la résine est conduite selon un traitement discontinu, sous agitation, à 0—40°C pendant 30 minutes à 72 heures.

11. Procédé selon l'une quelconque des revendications 1—10, dans lequel on effectue l'adsorption de l'enzyme sur la résine en faisant passer la solution d'enzyme à travers une colonne garnie de ladite résine.

12. Procédé selon l'une quelconque des revendications 1—11, dans lequel, avant le contact avec le polyaldéhyde, la résine contenant l'enzyme adsorbée est mise en contact avec une solution d'une substrat de l'enzyme dans laquelle la concentration du substrat au contact est de 0,1 mM—1,0 M.

13. Proocédé selon l'une quelconque des revendications 1—12, dans lequel la quantité de substrat utilisée est comprise dans l'intervalle de 10—1000 fois la quantité molaire de polyaldéhyde utilisée par la suite dans l'étape de réticulation.

14. Procédé selon l'une quelconque des revendications 1—13, dans lequel le traitement avec la solution du substrat est effectué sous agitation à 0—40°C pendant 10 minutes à 10 heures.

15. Procédé selon l'une quelconque des revendications précédentes, dans lequel le polyldéhyde utilisé dans l'étape de réticulation est un dialdéhyde aliphatique saturé ayant 2—10 atomes de carbone.

16. Procédé selon l'une quelconque des revendications précédentes, dans lequel l'étape de réticulation est effectuée par mise en contact de la résine contenant l'enzyme adsorbée avec une solution contenant le polyaldéhyde dans laquelle la concentration du polyaldéhyde au contact est de 1—100 mM.

17. Procédé selon l'une quelconque des revendications précédentes, dans lequel la quantité utilisée de polyaldéhyde est comprise dans l'intervalle allant de 0,001 mM à 1 mM par mg de l'enzyme, calculée en protéine.

18. Procédé selon l'une quelconque des revendications précédentes, dans lequel le traitement avec le polyaldéhyde est effectué à 0—40°C pendant 1 minute à 48 heures.

19. Procédé de régénération d'une composition à enzyme immobilisée, la composition étant produite par un procédé selon l'une quelconques des revendications 1—18, qui comprend le traitement de la composition à enzyme immobilisée, après usage, avec une solution aqueuse d'acide minéral contenant un sel alcalin de l'acide minéral à une concentration allant de 0,2 à 5 M, ce qui rompt les liaisons réticulant l'enzyme et permet la désorption de l'enzyme de la résine, et l'adsorption d'enzyme fraîche dans la résine par un procédé selon l'une quelconque des revendications 1—18.

20. Procédé selon la revendication 19, dans lequel la solution aqueuse d'acide utilisée pour rompre les liaisons de réticulation et désorber l'enzyme comprend un acide minéral à une concentration de 0,5—6 N.

21. Procédé selon la revendication 20 ou 21, dans lequel le sel alcalin est un sel de métal alcalin, de métal alcalino-terreux ou d'ammonium de l'acide sulfurique, de l'acide chlorhydrique ou de l'acide phosphorique, et l'acide minéral est l'acide sulfurique, l'acide chlorhydrique, ou l'acide phosphorique.

22. Procédé selon l'une quelconque des revendications 19—21, dans lequel l'enzyme est désorbée par contact avec ladite solution d'acide minéral, à une température comprise dans l'intervalle 40—95°C.